# EUROPEAN PATENT APPLICATION

(11) **EP 4 299 713 A1**
(43) Date of publication of application: **03.01.2024**
(21) Application number: 22759105.4
(22) Date of filing: 05.01.2022
(51) Int. Cl.: C12M 1/34, C12Q 1/06, C12Q 1/6813, C12Q 1/6837

(54) **MEASUREMENT METHOD AND MEASUREMENT SYSTEM**

(30) Priority: 26.02.2021 JP 2021030947
(71) Applicant: Yokogawa Electric Corporation, Tokyo 180-8750 (JP)
(72) Inventor: MIYAUCHI Yuki, Musashino-shi, Tokyo 180-8750 (JP); TAGUCHI Tomoyuki, Musashino-shi, Tokyo 180-8750 (JP)
(74) Representative: Winter, Brandl - Partnerschaft mbB
(86) International application number: PCT/JP2022/000141
(87) International publication number: WO 2022/181062

(57) **Abstract**

A measurement method for measuring an amount of microorganisms in a test sample, including: a step of concentrating and purifying the microorganisms in the test sample; a step of extracting a nucleic acid from the concentrated and purified microorganisms; a step of performing a hybridization reaction by adding to the extracted nucleic acid a hybridization reaction solution containing salts and surfactant necessary for the hybridization reaction of the extracted nucleic acid; a step of measuring a light emission intensity of the hybridized nucleic acid; a step of estimating the amount of the extracted nucleic acid based on the measured value of light emission intensity of the nucleic acid; and a step of calculating the amount of the microorganisms in the test sample based on the estimated amount of the extracted nucleic acid.

## Description

### CROSS-REFERENCE TO RELATED APPLICATION

The present application claims priority to and the benefit of Japanese Patent Application No. 2021-30947 filed February 26, 2021, the entire contents of which are incorporated herein by reference.

### TECHNICAL FIELD

The present disclosure relates to a measurement method and a measurement system for quantitative measurement of microorganisms.

### BACKGROUND

Conventional methods for performing gene amplification on a sample and detecting the gene after amplification are known (see, for example, Patent Literature (PTL) 1).

### CITATION LIST

### Patent Literature

PTL 1: JP 2015-43702 A

### SUMMARY

### (Technical Problem)

There is a demand for improved convenience when measuring microorganism amounts based on gene detection results.

It would be helpful to provide a measurement method and a measurement system capable of improving convenience in microorganism amount measurement.

### (Solution to Problem)

A measurement method according to at least one embodiment is a measurement method for measuring an amount of microorganisms in a test sample, comprising: a step of concentrating and purifying the microorganisms in the test sample; a step of extracting a nucleic acid from the concentrated and purified microorganisms; a step of performing a hybridization reaction by adding to the extracted nucleic acid a hybridization reaction solution containing salts and surfactant necessary for the hybridization reaction of the extracted nucleic acid; a step of measuring a light emission intensity of the hybridized nucleic acid; a step of estimating the amount of the extracted nucleic acid based on the measured value of light emission intensity of the nucleic acid; and a step of calculating the amount of the microorganisms in the test sample based on the estimated amount of the extracted nucleic acid. In this way, the amount of nucleic acid may be estimated without performing polymerase chain reaction (PCR) processing. Estimating the amount of nucleic acid without PCR processing reduces the influence of PCR reaction efficiency on the estimated amount of nucleic acid. The reduction of the influence of PCR reaction efficiency increases the accuracy of quantitative measurement of target microorganisms performed based on the estimated amount of nucleic acid. As a result, convenience in microorganism measurement is improved.

According to an embodiment, in the measurement method, in the step of performing the hybridization reaction, the extracted nucleic acid is subjected to the hybridization reaction at each of a plurality of spots of a microarray, and in the step of measuring the light emission intensity of the hybridized nucleic acid, the light emission intensity at each of the spots of the microarray is measured. In this way, many varieties of microorganisms are quantitatively and sensitively measured simultaneously. As a result, convenience in microorganism measurement is improved.

According to an embodiment, in the measurement method, in the step of performing the hybridization reaction, the nucleic acid is captured by a probe that is fixed on a solid phase surface of a spot of the microarray and modified with a fluorescent molecule. In this way, the amount of nucleic acid in the test sample brought into wet contact at each spot is measured as a luminance value of each spot. As a result, the accuracy of quantitative measurement of microorganisms is improved.

A measurement system according to at least one embodiment is for measuring an amount of microorganisms in a test sample. The measurement system comprises: a microorganism concentration apparatus operable to concentrate and purify the microorganisms in the test sample; a nucleic acid extraction apparatus operable to extract a nucleic acid from the microorganisms concentrated and purified by the microorganism concentration apparatus; a hybridization reaction apparatus operable to perform a hybridization reaction with the nucleic acid extracted by the nucleic acid extraction apparatus; and a nucleic acid detection apparatus operable to measure a light emission intensity of the nucleic acid hybridized to a probe by the hybridization reaction apparatus and estimate the amount of the extracted nucleic acid based on the measured value of light emission intensity of the nucleic acid. The nucleic acid detection apparatus causes a calculation device to calculate the amount of the microorganisms in the test sample based on the estimated amount of the extracted nucleic acid, and obtains a calculation result of the calculation device as a measurement result of the amount of the microorganisms. In this way, the amount of nucleic acid may be estimated without performing polymerase chain reaction (PCR) processing. Estimating the amount of nucleic acid without PCR processing reduces the influence of PCR reaction efficiency on the estimated amount of nucleic acid. The reduction of the influence of PCR reaction efficiency increases the accuracy of quantitative measurement of target microorganisms performed based on the estimated amount of nucleic acid. As a result, convenience in microorganism measurement is improved.

According to an embodiment, in the measurement system, the microorganism concentration apparatus concentrates and purifies microorganisms in the test sample by a negatively charged membrane method. In this way, it may be expected that the influence of clogging and the like is smaller and that larger sample volumes may be processed to increase the concentration rate, compared to typical filtering by a filter having a pore diameter smaller than a target microorganism.

### (Advantageous Effect)

The measurement method and the measurement system according to the present disclosure improve convenience in microorganism amount measurement.

### BRIEF DESCRIPTION OF THE DRAWINGS

In the accompanying drawings:
FIG. 1 is a flowchart illustrating example procedures of quantitative measurement by a qPCR method according to a comparative example;
FIG. 2 is a flowchart illustrating example procedures of pre-processing of multiplex qPCR according to a comparative example;
FIG. 3 is a flowchart illustrating example procedures of quantitative measurement by a microarray method according to a comparative example;
FIG. 4 is a flowchart illustrating example procedures of pre-processing of the microarray method according to a comparative example;
FIG. 5 is flowchart illustrating example procedures of a measurement method according to an embodiment; and
FIG. 6 is a block diagram illustrating an example configuration of a measurement system according to an embodiment.

### DETAILED DESCRIPTION

Embodiments of the present disclosure are described below with reference to the drawings. A measurement method according to an embodiment of the present disclosure is a method for measuring amounts of at least two varieties of microorganisms present in a test sample.

### (Comparative examples)

First, comparative examples are described to compare with the measurement method according to the present embodiment. A multiplex quantitative polymerase chain reaction (qPCR) method and a microarray method are described below as comparative examples of methods for simultaneous quantitative measurement of multiple microorganism amounts in a test sample.

### <Quantitative measurement by qPCR method>

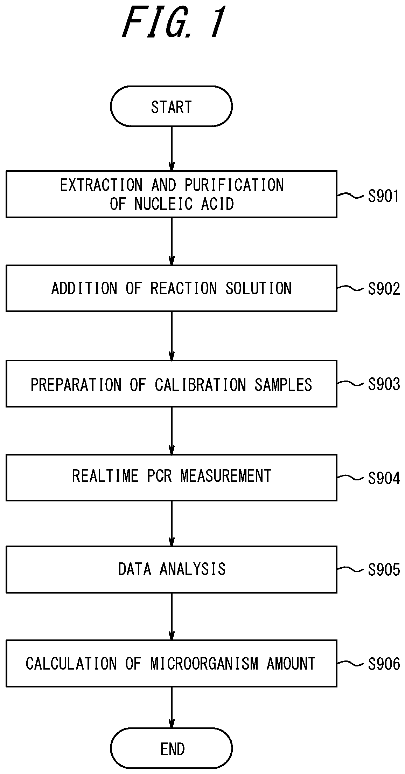

FIG. 1 illustrates example procedures for quantitative measurement by the qPCR method. Nucleic acid is extracted from the test sample and purified (step S901). A reaction solution is added to the purified sample (step S902). Aside from the test sample, samples for calibration are prepared by adding the reaction solution to a reference nucleic acid in known concentrations (step S903). Real-time PCR measurement of nucleic acid from the test sample and the reference nucleic acid is performed (step S904). Data analysis is performed to compare the Ct values of the real-time PCR measurement of the reference nucleic acid at the known concentrations with the Ct values of the nucleic acid from the test sample (step S905). Based on the nucleic acid content of the test sample estimated by the data analysis, the microorganism amounts are calculated (step S906).

Preparation of the reference nucleic acid of the known concentrations for calibration in step S903 is performed, for example, by preparing a 1/10 dilution series or by preparing a range that includes the nucleic acid concentration from the test sample. Preparation of the reference nucleic acid of the known concentrations for calibration needs to be performed so that the concentrations are equal to or more than a lower detection limit of the qPCR apparatus and the concentration range is prepared in advance according to the specification of the apparatus so that quantifiability is not lost due to reaction inhibition.

The nucleic acid from the test sample is sampled in a dilution series so that the concentrations are equal to or more than the lower detection limit of the qPCR apparatus and the concentration range is prepared in advance according to the specification of the apparatus so that quantifiability is lost due to reaction inhibition.

In the qPCR reaction, an intercalator (a fluorescent reagent that non-specifically stains double-stranded nucleic acid) or a fluorescent nucleic acid probe is used to measure nucleic acid amplification. Fluorescent nucleic acid probes are also referred to as TaqMan probes.

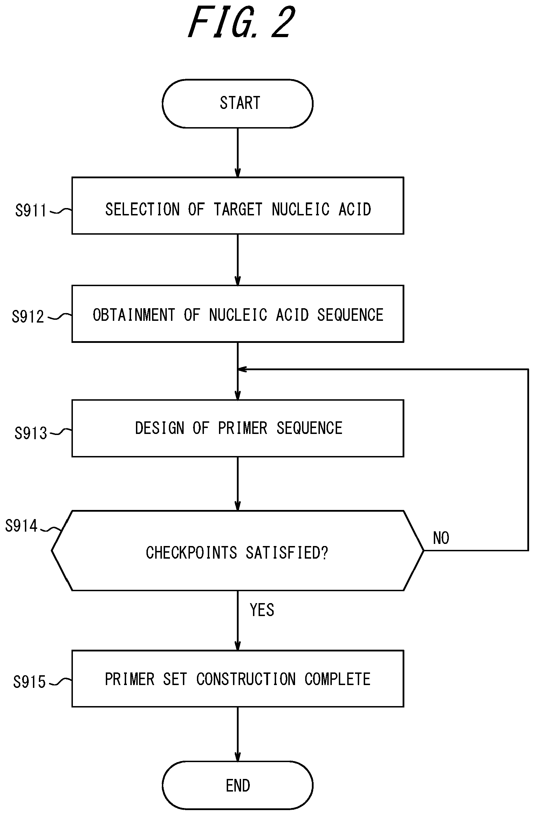

FIG. 2 illustrates example procedures for constructing a primer set as pre-processing required to perform multiplex qPCR for simultaneous detection of multiple nucleic acids based on the qPCR method. First, a nucleic acid specific to a target microorganism is selected as a target nucleic acid from among genes or other nucleic acids of the target microorganism (step S911). Next, a nucleic acid sequence of the target nucleic acid selected in step S911 is obtained (step S912). The nucleic acid sequence of the target nucleic acid may be obtained from a nucleic acid sequence contained in a publicly available nucleic acid sequence database or the like, or may be obtained by nucleic acid sequencing or other techniques.

Next, primer sequences necessary to amplify the target nucleic acid are designed based on the target nucleic acid sequence obtained in step S912 (step S913). The primer sequences are designed so that Tm values are equivalent between the forward and reverse primers. The Tm value, also referred to as the melting temperature, represents the temperature at which the primer forms a double strand with the target sequence 50 % of the time. Further, a PCR amplification reaction is performed to confirm that the target nucleic acid can be efficiently amplified by the designed primers. The PCR amplification reaction is performed in a buffer solution containing the template nucleic acid, bases, and elongase required for the reaction, as well as salts required for the enzymatic reaction, all based on publicly available information. Further, the PCR amplification reaction is controlled by a temperature cycle appropriate for the enzyme used, consisting of three steps: annealing, extension, and thermal denaturation. The temperature cycle may consist of two steps where extension and thermal denaturation are performed at the same temperature.

Following the PCR amplification reaction, whether checkpoints have been satisfied is determined by gel electrophoresis or the like, to confirm that PCR amplification products have been produced (step S914). Checkpoints include amount of amplification, amplification length, the presence or absence of amplification of nonspecific products, the presence or absence of primer dimer formation, and the like. When checkpoints do not satisfy requirements, an improvement or redesign of the primers is performed. Then confirmation is required that the improved or redesigned primers are capable of amplifying the target nucleic acid. Primers designed to satisfy the checkpoint conditions are determined as individual stand-alone primers.

Further, when multiple varieties of nucleic acids are to be detected simultaneously, individual stand-alone primers must be constructed for each variety of nucleic acid. After constructing individual stand-alone primers for all target nucleic acids, PCR amplification is performed on a primer set that is a mix of the individual stand-alone primers constructed for each variety of nucleic acid. Then, to confirm that PCR amplification with the primer set has produced PCR amplification products, whether or not checkpoints have been satisfied is determined. The checkpoints for PCR amplification with the primer set may be the same as those for PCR amplification with individual stand-alone primers.

When PCR amplification with the primer set does not satisfy the checkpoint conditions, due to the production of a nonspecific product for example, (step S914: NO), processing returns to step S913 and the individual stand-alone primer is redesigned. When the checkpoint conditions are satisfied (step S914: YES), the construction of a primer set in which nonspecific products do not occur is completed (step S915).

The qPCR method described above has the following problems. Each target nucleic acid species is detected at a different wavelength, and therefore a limitation of fluorescence wavelength limits the number of species that are simultaneously detectable to about six. Increased development costs and time spent on design, prototyping, and evaluation performed to create primers that do not produce nonspecific PCR amplification products. Increased development costs and time spent to confirm the cross-reactivity of multiple primers, especially in multiplex qPCR. The increase in the number of reactions due to the simultaneous use of multiple calibration reference samples in a dilution series increases the cost of consumables such as reagents and the time and labor required for the reactions. Different PCR reaction efficiencies for each target nucleic acid require different reference samples for calibration for each target nucleic acid. Therefore, the number of reactions is high. The increase in the number of reactions increases the cost of consumables such as reagents and the like, and labor. For example, when five varieties of bacteria are to be detected, 25 different reactions are required, combining five test samples and reference samples for five-point calibration. Increased development costs and time spent to add target microorganisms after a primer set has been constructed. The presence or absence of amplification of nonspecific products due to contamination of unknown specimens or the generation of primer dimers will result in false positive detection or reduced quantifiability. PCR reaction efficiency is altered by inhibitors of the enzymatic reaction in the sample. Changes in PCR reaction efficiency reduce the quantifiability of qPCR.

### <Quantitative measurement by microarray method>

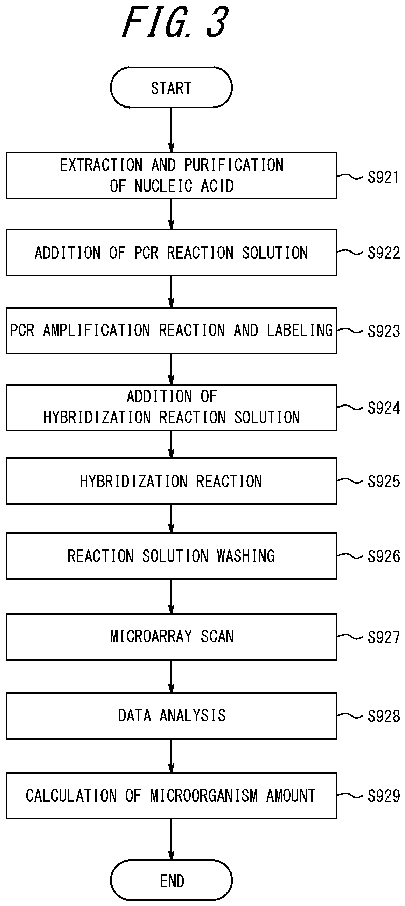

FIG. 3 illustrates example procedures for quantitative measurement by the microarray method. Nucleic acid is extracted from the test sample and purified (step S921). PCR reaction solution is added to the purified sample (step S922). The sample is subjected to PCR amplification reaction and labeled (step S923). Hybridization reaction solution is added to the PCR amplified sample (step S924). Nucleic acid from the test sample is subjected to hybridization reaction at probes on the microarray (step S925). The reaction solution is removed by washing (step S926). The probes on the microarray are scanned to obtain images of the probes subjected to the hybridization reaction (step S927). Data analysis is performed to quantify the light intensity of the label hybridized to the probes and compare to the light intensity of the label with a reference nucleic acid of known concentration obtained in advance (step S928). Based on the nucleic acid amount estimated by data analysis, the microorganism amount is calculated (step S929).

In order to subject the sample to the PCR amplification reaction in step S923, a primer set construction process is required, as mentioned in the description of the qPCR method. Typically, in primers used in microarrays, labels such as fluorescent molecules are used so that PCR amplification products are labeled.

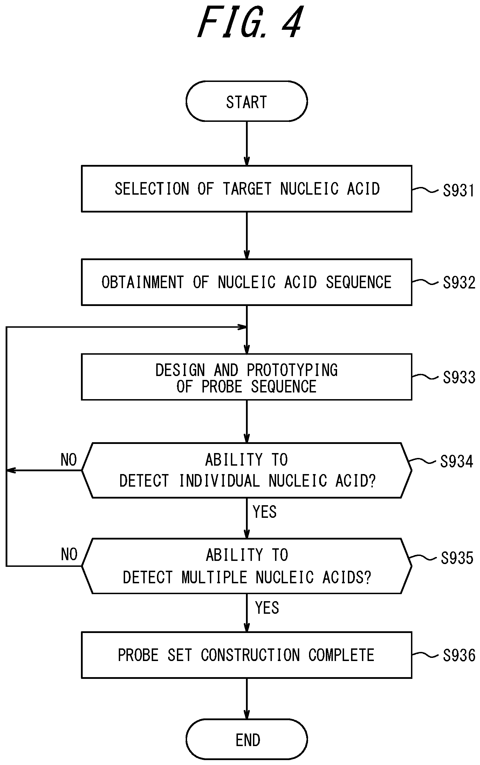

FIG. 4 illustrates example probe construction procedures as preprocessing required for simultaneous detection of multiple nucleic acids based on the microarray method. First, a target nucleic acid specific to a target microorganism is selected from among genes or other nucleic acids of the target microorganism (step S931). Next, a nucleic acid sequence of the selected target nucleic acid is obtained (step S932). Next, a probe sequence necessary to detect the target nucleic acid from the nucleic acid sequence is designed and prototyped (step S933). By subjecting the prototyped probe to a hybridization reaction against an individual nucleic acid, whether the prototyped probe has a detection ability is determined (step S934). When the prototyped probe is not capable of detection for an individual nucleic acid (step S934: NO), processing returns to step S933 and the probe sequence is redesigned. When the prototype probe has a detection ability for an individual nucleic acid (step S934: YES), whether the prototype probe has a detection ability for a sample containing multiple nucleic acids is determined (step S935). When the prototyped probe is not capable of detection for a sample containing multiple nucleic acids (step S935: NO), processing returns to step S933 and the probe sequence is redesigned. When the prototyped probe is capable of detection for a sample containing multiple nucleic acids (step S935: YES), the probe set construction is complete (step S936).

Checkpoints in the determination of steps S934 and S935 include, for example, the light intensity of the label hybridized and fixed to the probe. Checkpoints include, but are not limited to, checkpoints for elimination of false positive factors such as checking whether a nucleic acid other than the target nucleic acid is not hybridized and fixed, checking whether there are no cross reactions where primers are hybridized and fixed, and the like.

In obtaining the nucleic acid sequence in step S932, a corresponding nucleic acid sequence may be obtained from a publicly available nucleic acid sequence database or the like. Further, a target nucleic acid sequence may be obtained independently by nucleic acid sequencing or other methods. In designing the probe array in step S933, the probe length is designed so that the Tm values of each probe in the probe set are equivalent. The hybridization reactions performed in steps S934 and S935 are carried out in buffer solution with salts and surfactants required for the reaction based on known information. Further, the hybridization reactions are carried out by incubating at a temperature suitable for the Tm value of the designed probe.

Another possible method is to measure targets using an apparatus for nucleic acid sequence measurement (DNA chip) in which fluorescent probes to which fluorescent molecules are attached and quencher probes to which quencher molecules that quench the fluorescence of the fluorescent molecules are attached are provided as detection probes. This method makes measuring targets possible without adding fluorescent molecules to the target and washing the DNA chip (washing to remove uncollected targets and the like).

The microarray method described above has the following problems. In microarray detection including PCR processing, increased development costs and time spent on design, prototyping, and evaluation for hybridization fixation of a nucleic acid other than the target nucleic acid, or for creating probes without cross-reactivity in which primers are hybridized and fixed. In microarray detection including PCR processing, different PCR reaction efficiencies for each target nucleic acid affect quantifiability of microorganism amount calculation. In microarray detection including PCR processing, PCR reaction efficiency is altered by inhibitors of the enzymatic reaction contained in the sample. Changes in PCR reaction efficiency reduce the quantifiability of the microorganism amount calculation. The washing process results in loss of hybridized target nucleic acid or false positives due to contamination of adjacent samples on the microarray. The occurrence of false positives reduces quantifiability. The sensitivity of microarrays is low relative to qPCR. Therefore, the lower limit of detection is high when PCR processing is excluded.

As described above, various methods may be considered as comparative examples of methods for measuring microorganism amounts in a test sample. The various methods have problems, as described above. In view of the problems described above, the measurement method according to the present disclosure is described below.

### (Embodiment according to the present disclosure)

The measurement method according to an embodiment of the present disclosure is able to quantitatively and sensitively measure many varieties of microorganisms simultaneously by performing microarray detection without PCR processing. Further, the measurement method according to the embodiment of the present disclosure is able to measure target microorganisms with high sensitivity by performing microarray detection without PCR processing that includes the concentration and purification processing of the test sample. Further, the measurement method according to the embodiment of the present disclosure is able to quantitatively measure target microorganisms by performing microarray detection without PCR processing, thereby eliminating the influence of PCR reaction efficiency. Further, the measurement method according to the embodiment of the present disclosure is able to quantitatively measure target microorganisms by performing microarray detection without labeling, thereby eliminating the influence of labeling efficiency. Further, the measurement method according to the embodiment of the present disclosure is able to measure without increasing the cost of testing compared to qPCR, even when the number of target microorganism varieties increases, by performing microarray detection. Further, the measurement method according to the embodiment of the present disclosure decreases primer development costs and time by performing microarray detection without PCR processing. Further, the measurement method according to the embodiment of the present disclosure decreases the cost of consumables such as PCR reaction reagents and decreases labor by performing microarray detection without PCR processing. Further, the measurement method according to the embodiment of the present disclosure is able to reduce development costs by performing microarray detection without PCR processing, as a result of eliminating cross-reactions with primers in probe development and thereby facilitating development. Further, the measurement method according to the embodiment of the present disclosure is able to reduce the risk of false positives and the risk of reduced quantifiability due to washing by performing microarray detection without washing processing.

### (Example procedures of measurement method)

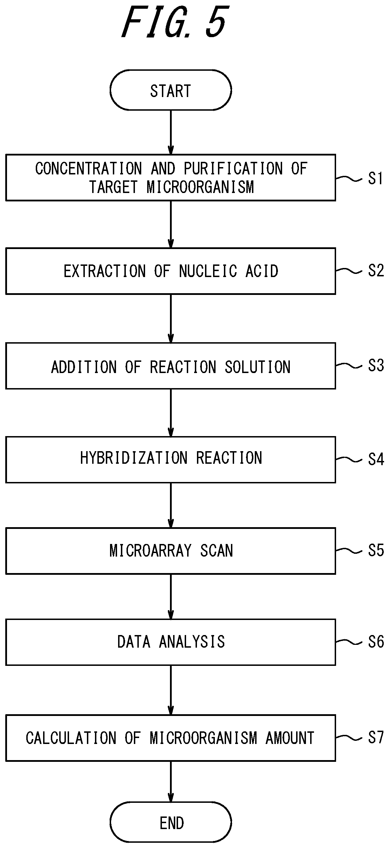

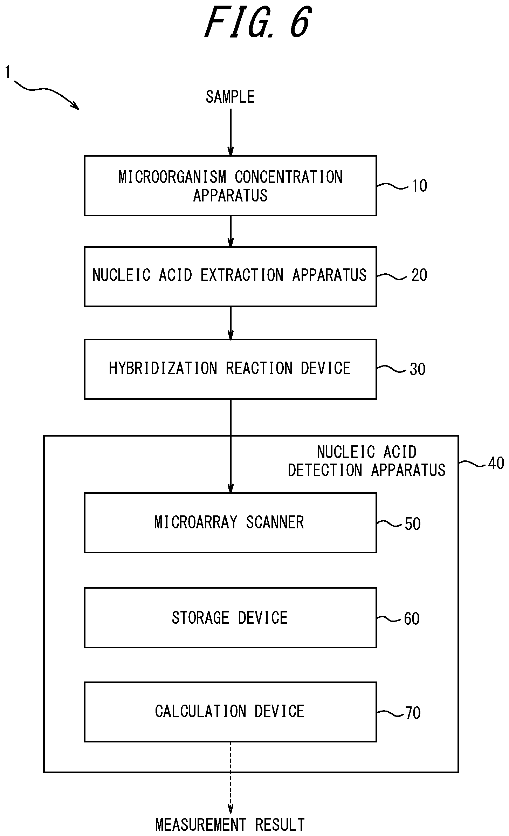

The measurement method according to the embodiment of the present disclosure is performed according to the procedures of the flowcharts illustrated in FIG. 5 and FIG. 6. First, processing of the example of microarray detection is described with reference to FIG. 5.

The test sample is concentrated and purified to recover target microorganisms from the test sample (Step S1). Nucleic acid is extracted from the concentrated and purified target microorganisms (Step S2). Hybridization reaction solution is added to the sample from which nucleic acid has been extracted (Step S3). The nucleic acid extract to which the hybridization reaction solution is added is subjected to the hybridization reaction (Step S4). In the procedure of step S4, the nucleic acid extract is brought into wet contact with the probes fixed in spots on the microarray. The microarray is microarray scanned to obtain a fluorescence image of each probe of the microarray after the hybridization reaction (step S5). The fluorescence image is obtained to measure the light emission intensity of hybridized nucleic acid. Data analysis is performed to compare the luminance value of each spot obtained from the fluorescence image with the luminance value of the spot with a reference nucleic acid of known concentrations obtained in advance (step S6). The luminance value corresponds to the measured value of light emission intensity of the nucleic acid. The amount of microorganisms is calculated based on the amount of nucleic acid estimated by the data analysis (step S7). The amount of nucleic acid estimated by the data analysis corresponds to the estimated amount of the extracted nucleic acid.

As illustrated in FIG. 6, a measurement system 1 for executing the measurement method according to the present embodiment includes a microorganism concentration apparatus 10, a nucleic acid extraction apparatus 20, a hybridization reaction apparatus 30, and a nucleic acid detection apparatus 40. In FIG. 6, solid arrows joining component elements represent the flow of the sample containing target microorganisms or target nucleic acid. The measurement system 1 includes, in order from upstream to downstream sample flow, the microorganism concentration apparatus 10, the nucleic acid extraction apparatus 20, the hybridization reaction apparatus 30, and the nucleic acid detection apparatus 40. In FIG. 6, the dashed arrow represents a measurement result being output from the nucleic acid detection apparatus 40.

The microorganism concentration apparatus 10 concentrates and purifies target microorganisms by a negatively charged membrane method. The test sample to be concentrated and purified is a liquid containing microorganisms or a solid on which microorganisms are dispersed in a liquid. Microorganisms in water such as bacteria and viruses have an isoelectric point in a region where surface charge is weakly acidic, are negatively charged in neutral to alkaline water, and positively charged in acidic water. The negatively charged membrane method is able to use this property to capture microorganisms from water. The negatively charged membrane method, when compared to typical filtering by a filter having a pore diameter smaller than a target microorganism, may be expected to have less of a clogging effect, and may be expected to increase concentration rate by processing larger sample volumes, among other benefits.

The negatively charged film method may be performed based on a method described in the following non-patent literature (NPL). NPL: H. Katayama et al, "Development of a Virus Concentration Method and Its Application to Detection of Enterovirus and Norwalk Virus from Coastal Seawater", Applied and Environmental Microbiology, 2002, Vol. 68, p. 1033-1039

The nucleic acid extraction apparatus 20 is able to perform extraction and purification by chemical or physical processes using a typically applied commercially available kit. A chemical nucleic acid extraction process may be performed by the action of phenol, enzymes that degrade microorganism cell membranes, surfactants, salts to stabilize nucleic acids, chelating agents that inactivate nucleases and prevent degradation of nucleic acids, buffers containing pH buffers, and the like, or by adding heat treatment and the like to these actions. A physical nucleic acid extraction process may be performed by homogenizer pestles, ultrasonic devices, freeze-fracturing devices, bead mills, high-pressure homogenizers, and the like. An extracted nucleic acid may be purified by phenol chloroform extraction, chloroform extraction, ethanol precipitation, a spin column method that is also available as a commercial kit, a magnetic bead method, and the like, as required. Hybridization reaction solution is added to the nucleic acid extract. The hybridization reaction solution contains salts and surfactants necessary for hybridization of nucleic acids. For example, a buffer solution having a final concentration of 5 x saline-sodium citrate (SSC) or the like may be used.

The hybridization reaction apparatus 30 subjects the nucleic acid extract to which hybridization reaction solution is added to a hybridization reaction while the probes are in wet contact with the solution. In the hybridization reaction apparatus 30, the nucleic acid extract is inserted into a chamber or the like configured to hold samples that are installed one-to-one with one or more blocks, which are each a collection of spots of multiple probes set on a microarray, and the nucleic acid extract is brought into wet contact with the blocks on the microarray.

The microarray has a plurality of spots. Probes that are modified with fluorescent molecules are fixed on spot solid phase surfaces, in one-to-one correspondence. The nucleic acid extract is brought into wet contact with the probes fixed to spots on the microarray. Nucleic acid in the nucleic acid extract is captured by hybridizing with probes in wet contact. In other words, the hybridization reaction apparatus 30 causes nucleic acid to be captured by probes by the hybridization reaction.

The hybridization reaction may be promoted by incubating the probes at a temperature set appropriately for the Tm value of the probes. Further, the hybridization reaction is also promoted by shaking or rotating the sample during incubation to assist in the diffusion of nucleic acid in the sample. The hybridization reaction may be promoted by incubation under light-shielded conditions at, for example, 60 °C for 30 mins.

The nucleic acid detection apparatus 40 includes a microarray scanner 50, a storage device 60, and a calculation device 70.

The microarray scanner 50 obtains fluorescence images of the spots where microarray probes are fixed. The microarray scanner 50 outputs the fluorescence image as a detection signal to the storage device 60 or the calculation device 70. When the microarray is a transparent substrate, the microarray scanner 50 scans the spots from the opposite side to the microarray spot surface after the hybridization reaction. When the microarray is an opaque substrate, the microarray scanner 50 scans the spots from the bottom of the chamber after the hybridization reaction. In this case, a surface parallel to the microarray in the chamber mentioned above must be transparent.

The calculation device 70 calculates spot luminance values based on the fluorescence image signal output from the microarray scanner 50. The spot luminance values may be calculated from an area designation on the image by image recognition of the spots and the average luminance values of each pixel of the spots. Further, the calculation device 70 estimates nucleic acid concentration of the test sample from luminance values of spots and a calibration curve of nucleic acid concentration for a reference nucleic acid of known concentrations obtained in advance after an arbitrary hybridization reaction time. The calculation device 70 may include any calculation element or calculation module capable of calculating the luminance values of a fluorescence image. The spot luminance values may be converted to any value, such as light intensity, based on the device specifications of the microarray scanner 50. The spot luminance values may be measured as luminance values at multiple time points in the hybridization reaction between a target nucleic acid and probes performed by the hybridization reaction apparatus 30. Luminance values during progress of a hybridization reaction may increase as the reaction proceeds. The increase in luminance values stops when the hybridization reaction between the target nucleic acid and the probes reaches equilibrium. Quantification in a state where the luminance values have stopped increasing becomes impossible. By obtaining luminance values at multiple time points, the calculation device 70 may estimate nucleic acid concentration from the luminance values before the hybridization reaction reaches equilibrium when the target nucleic acid is highly concentrated.

Further, the calculation device 70 may quantify the target nucleic acid by a calibration curve based on the luminance values at multiple time points. The calculation device 70 may quantify the target nucleic acid based on the raw data of the image or the signal obtained by filtering or calculation processing of the image, without calculating the luminance values. In this case, the quantification of the target nucleic acid based on the signal may be performed as obtaining calculated results from a multivariate analysis or a machine learning quantification model. The quantification of the target nucleic acid may be fed back into the quantification model by obtaining reference values at the same time as the quantification results from the present method.

The calculation device 70 may calculate the amount of microorganisms from target nucleic acid quantification results with reference to values from previously published literature for nucleic acid numbers per cell. The calculation device 70 may obtain a calibration curve of spot luminance values and microorganism concentration when the measurement method of the present disclosure is performed in advance on a reference microorganism of known concentrations for quantitative model construction, and calculate the microorganism concentration of the test sample based on the calibration curve. The calculation of amount of microorganisms may be performed as a multivariate analysis or by machine learning to obtain calculated results from a quantitative model. The calculation of the amount of microorganisms may be fed back into the quantitative model by obtaining reference values at the same time as the quantitative results from this method.

Calculation of the amount of microorganisms is not limited to the calculation device 70, and may be calculated by a device external to the calculation device 70. That is, the nucleic acid detection apparatus 40 may cause the calculation device 70 that is internal or an external device to calculate the amount of microorganisms and obtain the calculation results as the measured amount of microorganisms present.

The storage device 60 stores the image signals from the microarray scanner 50 as appropriate and outputs to the calculation device 70. The storage device 60 is able to store quantification results of target nucleic acids and microorganisms from the calculation device 70, as appropriate.

The measurement system 1 may further include a display device. The display device may obtain and display target nucleic acid or microorganism quantification results from the calculation device 70 or the storage device 60.

The probes used may be the probes described in PTL 1 (JP 2015-43702 A). Specifically, the probes may be fluorescent probes to which fluorescent molecules are added and quencher probes to which quencher molecules are added. The fluorescent probes and the quencher probe are combined so that fluorescence of the fluorescent molecules is quenched by the quencher molecules. When the target to be measured is added to a probe, hybridization dissolves the binding between the fluorescent probe and the quencher probe, causing the probe to emit fluorescence. Fluorescent probes may be optionally labeled with fluorescent dyes or other labels. Excitation and fluorescence detection wavelengths of the microarray scanner 50 are selected based on absorption and fluorescence wavelength properties of the fluorescent dye used in the fluorescent probes.

The microarray scanner 50 is not limited to the embodiment described above, and may include a fluorescence microscope, confocal microscope, evanescent fluorescence detection device, thin-film oblique illumination microscope, light sheet microscope, structured illumination microscope, multiphoton excitation microscope, and the like to detect fluorescence from spots on the microarray.

As described above, according to the measurement method of the present embodiment, many varieties of microorganisms are quantitatively and sensitively measured simultaneously by microarray detection. Further, target microorganisms are measured with high sensitivity by microarray detection that includes concentration and purification processing of a test sample. Further, the influence of PCR reaction efficiency is reduced by microarray detection excluding PCR processing. The reduced influence of PCR reaction efficiency increases the accuracy of quantitative measurement of target microorganisms. Further, microarray detection is less likely to increase test costs than qPCR even when the number of target microorganism species increases. Further, microarray detection excluding PCR processing reduces the cost and time spent on primer development. Further, microarray detection excluding PCR processing reduces the cost of consumables such as reagents for PCR reactions and the labor involved in PCR reactions. Further, microarray detection excluding PCR processing eliminates cross-reactions with primers for probe development. The absence of cross-reactions facilitates development and reduces development costs. Further, the influence of labeling efficiency is reduced by microarray detection excluding labeling processing. The reduced influence of labeling efficiency increases the accuracy of quantitative measurements of target microorganisms. Further, microarray detection without washing processing reduces the risks of false positives and loss of quantifiability due to washing.

According to a comparative example, the amount of microorganisms in microarray detection is calculated from the final hybridized nucleic acid content based on the following processes: A: microorganism recovery efficiency, B: nucleic acid extraction and purification efficiency, C: PCR reaction efficiency, D: hybridization efficiency, E: loss due to washing, and F: reference value for nucleic acid amount per cell. PCR reaction and washing not being performed in the measurement method according to the present embodiment reduces the influence of C: PCR reaction efficiency, which causes a large efficiency change depending on the sample, and E: loss due to washing, which tends to cause differences due to technique. In this way, quantitative measurement accuracy is increased.

As a result of the above, the measurement method according to the present embodiment improves convenience in microorganism measurement.

### (Example of application)

The measurement method according to the present embodiment may be applied as follows. For example, the measurement method according to the present embodiment may be used for dry image measurement in fluorescence amount measurement, biochip fluorescence amount solution observation, real-time observation in continuous reactions, and the like. As specific examples, the measurement method according to the present embodiment may be used for nucleic acid analysis of bacterial species identification, cancer genes, animal and plant identification, intestinal bacteria testing, and the like.

Further, the measurement method according to the present embodiment may be horizontally deployed to other fields such as water supply, food, drinking water, and the like. In such cases, the sampling volume of a test sample may be set according to the microorganism amount for each application.

The calculation device 70 is able to estimate the nucleic acid concentration of a test sample based on the luminance values of spots and a calibration curve of nucleic acid concentration for a reference nucleic acid of known concentrations obtained in advance after an arbitrary hybridization reaction time. Methods for estimating nucleic acid concentration are not limited to this. For example, when the number of target nucleic acids and labels thereof are in a quantitative ratio, such as 1:1, the luminance values of spots correlates with the number of hybridized nucleic acids. Similarly, the luminance values of spots correlates 1:1 with the number of hybridized nucleic acids when the probes described in PTL 1 are used. The calculation device 70 is able to quantify nucleic acid concentration by quantitatively obtaining the luminance values of spots and comparing to the luminance values of reference spots having constant numbers of labeled molecules. Further, the calculation device 70 is able to calculate the number of hybridized nucleic acids from the light intensities of spots and theoretical light intensities of labels by calculating the luminance values of spots as absolute values of light intensity. Calculation of the absolute values of light intensity requires setting of parameters for each device, and may be done by standardizing a sensor or optical system using a reference light source or the like and assigning light intensity relative to sensor luminance value.

The present disclosure is not limited to the configurations specified in the embodiments described above, and various modifications are possible without departing from the scope of the claims. For example, the functions and the like included in each step may be reconfigured as long as no logical inconsistency arises. Multiple steps may be combined into one, and a single step may be split into more than one.

### REFERENCE SIGNS LIST

1 Measurement system (10: microorganism concentration apparatus, 20: nucleic acid extraction apparatus, 30: hybridization reaction apparatus, 40: nucleic acid detection apparatus, 50: microarray scanner, 60: storage device, 70: calculation device)

## Claims

1. A measurement method for measuring an amount of microorganisms in a test sample, comprising:
a step of concentrating and purifying the microorganisms in the test sample;
a step of extracting a nucleic acid from the concentrated and purified microorganisms;
a step of performing a hybridization reaction by adding to the extracted nucleic acid a hybridization reaction solution containing salts and surfactant necessary for the hybridization reaction of the extracted nucleic acid;
a step of measuring a light emission intensity of the hybridized nucleic acid;
a step of estimating the amount of the extracted nucleic acid based on the measured value of light emission intensity of the nucleic acid; and
a step of calculating the amount of the microorganisms in the test sample based on the estimated amount of the extracted nucleic acid.

2. The measurement method according to claim 1, wherein, in the step of performing the hybridization reaction, the extracted nucleic acid is subjected to the hybridization reaction at each of a plurality of spots of a microarray, and
in the step of measuring the light emission intensity of the hybridized nucleic acid, the light emission intensity at each of the spots of the microarray is measured.

3. The measurement method according to claim 2, wherein, in the step of performing the hybridization reaction, the nucleic acid is captured by a probe that is fixed on a solid phase surface of a spot of the microarray and modified with a fluorescent molecule.

4. A measurement system for measuring an amount of microorganisms in a test sample, comprising:
a microorganism concentration apparatus operable to concentrate and purify the microorganisms in the test sample;
a nucleic acid extraction apparatus operable to extract a nucleic acid from the microorganisms concentrated and purified by the microorganism concentration apparatus;
a hybridization reaction apparatus operable to perform a hybridization reaction by adding to the nucleic acid extracted by the nucleic acid extraction apparatus a hybridization reaction solution containing salts and surfactant necessary for the hybridization reaction of the extracted nucleic acid; and
a nucleic acid detection apparatus operable to measure a light emission intensity of the nucleic acid hybridized to a probe by the hybridization reaction apparatus and estimate the amount of the extracted nucleic acid based on the measured value of light emission intensity of the nucleic acid, wherein
the nucleic acid detection apparatus causes a calculation device to calculate the amount of the microorganisms in the test sample based on the estimated amount of the extracted nucleic acid, and obtains a calculation result of the calculation device as a measurement result of the amount of the microorganisms.

5. The measurement system according to claim 4, wherein the microorganism concentration apparatus concentrates and purifies microorganisms in the test sample by a negatively charged membrane method.

## Amended claims

### Amended claims under Art. 19.1 PCT

1. (Amended) A measurement method for measuring an amount of microorganisms in a test sample, comprising:
a step of concentrating and purifying the microorganisms in the test sample;
a step of extracting a nucleic acid from the concentrated and purified microorganisms;
a step of performing a hybridization reaction by adding to the extracted nucleic acid a hybridization reaction solution containing salts and surfactant necessary for the hybridization reaction of the extracted nucleic acid such that the extracted nucleic acid is capturable at spots of a microarray where probes modified with fluorescent molecules are fixed;
a step of measuring a light emission intensity at the spots of the microarray to measure a light emission intensity of the hybridized nucleic acid;
a step of estimating the amount of the extracted nucleic acid based on the measured value of light emission intensity of the nucleic acid; and
a step of calculating the amount of the microorganisms in the test sample based on the estimated amount of the extracted nucleic acid.

2. (Amended) The measurement method according to claim 1, wherein, in the step of estimating the amount of the extracted nucleic acid, concentration of the nucleic acid in the test sample is estimated based on luminance values of spots for a reference nucleic acid of known concentrations obtained in advance after an arbitrary hybridization reaction time and a calibration curve of nucleic acid concentration.

3. (Canceled)

4. (Amended) A measurement system for measuring an amount of microorganisms in a test sample, comprising:
a microorganism concentration apparatus operable to concentrate and purify the microorganisms in the test sample;
a nucleic acid extraction apparatus operable to extract a nucleic acid from the microorganisms concentrated and purified by the microorganism concentration apparatus;
a hybridization reaction apparatus operable to perform a hybridization reaction by adding to the nucleic acid extracted by the nucleic acid extraction apparatus a hybridization reaction solution containing salts and surfactant necessary for the hybridization reaction of the extracted nucleic acid such that the extracted nucleic acid is capturable at spots of a microarray where probes modified with fluorescent molecules are fixed; and
a nucleic acid detection apparatus operable to measure a light emission intensity of the nucleic acid hybridized to a probe by the hybridization reaction apparatus and estimate the amount of the extracted nucleic acid based on the measured value of light emission intensity of the nucleic acid, wherein
the nucleic acid detection apparatus causes a calculation device to calculate the amount of the microorganisms in the test sample based on the estimated amount of the extracted nucleic acid, and obtains a calculation result of the calculation device as a measurement result of the amount of the microorganisms.

5. The measurement system according to claim 4, wherein the microorganism concentration apparatus concentrates and purifies microorganisms in the test sample by a negatively charged membrane method.
